Europäisches Patentamt

(19) European Patent Office

Office européen des brevets

(11) EP 0 855 181 A2

(12) EUROPEAN PATENT APPLICATION

(43) Date of publication:
29.07.1998 Bulletin 1998/31

(51) Int. Cl.$^6$: A61K 31/195

(21) Application number: 98101299.0

(22) Date of filing: 26.01.1998

(84) Designated Contracting States:
AT BE CH DE DK ES FI FR GB GR IE IT LI LU MC NL PT SE
Designated Extension States:
AL LT LV MK RO SI

(30) Priority: 28.01.1997 GB 9701674

(71) Applicant: Novartis Nutrition AG
3001 Bern (CH)

(72) Inventor: König, Wolfgang
45657 Recklinghausen (DE)

(74) Representative:
Schubert Santana, Isabelle et al
Novartis AG,
Patent & Trademark Dept.,
Lichtstrasse 35
4002 Basel (CH)

(54) **Medicament or nutritional formulation for immune-modulation with amino acids**

(57)    The present invention provides the use of glycine, L-alanine and/or L-serine, in free amino acid form and/or in physiologically acceptable salt form in the preparation of a medicament or nutritional formulation with immune-modulating effect.

IL-10 release from human mononuclear cells      Fig. 1

preincubation with
glycine: 24h

GM-CSF: 10ng/ml

SEB: 10ng/ml

# EP 0 855 181 A2

**Description**

The present invention relates to the use of specific amino acids as immune-modulators in the natural defence against infection and inflammatory processes.

A major determinant of survival in patients with advanced viral or bacterial infection, or following severe trauma or burn, is the combination of clinical signs termed the systemic inflammatory response syndrome (SIRS). SIRS is characterized by hypotension, tachypnea, hypo- or hyperthermia and leukocytosis as well as other clinical signs and symptoms, including a depression in myocardial contractile function.

Pathophysiologically, SIRS is characterized by the activation of several groups of cell (monocytes/macrophages, polymorphonuclear granulocytes (PMNs), and endothelial cells) and by the release of inflammatory mediators (cytokines and others) such as tumor necrosis factor (TNF), interferon-$\gamma$ (IFN-$\gamma$), interleukin-10 (IL-10) and other interleukins (IL) such as IL-1, IL-6, IL-8 and IL-12.

Interleukin-6 as well as TNF-$\alpha$ are proinflammatory cytokines whereas interleukin-10 is an antiinflammatory cytokine which seems to regulate the cytokine cascade. The cytokines interferon-$\gamma$ as wells as interleukin-12 are of extraordinary importance in the microbial defence.

Recent investigations indicate that a lot of studies in the field of immunology/sepsis were carried out with oversimplified or wrong models and/or approaches. For example the sepsis mouse model using galactosamine renders the model oversenstitive to LPS, obviously this is clinically irrelevant and does not correspond to the human situation. Galactosamine modulates cellular reactivity of immune effective cells in sensitive mice in a different manner than in human cells. Experimental studies in the past in various animal models suggested a key role for TNF as mediator in sepsis. These results led to the assumption that anti-TNF antibodies as therapeutical regimen might help to overcome threatening sepsis. However, application of anti-TNF antibodies already in animal models rather showed an impairment of host defence with the facilitation of opportunistic infections. Studies in humans presented on various occasions also proved that anti-TNF strategy might be a rather doubtful regimen to treat sepsis. There is indication that anti-TNF antibodies might he useful in treating locally occurring inflammatory diseases. However, more trials are required. Certainly the impairment of host defence and the resulting disease process of sepsis and/or SIRS are a complex phenomenon based on multiple inducing stimuli, inflammatory mediators, cells and their signal transduction cascades.

A particular disease syndrome cannot be induced by a single irritant and gain in scientific knowledge has diminished the importance of single mediators. More emphasis is placed on interactive elements and on endogenously starting the mediator cascade.

It has now surprisingly been found that, *inter alia*, glycine is a potent immune-modulator in the natural defence against infection and inflammatory processes and as such is has a protective effect against SIRS.

The present invention therefore provides for the use of glycine, L-alanine and/or L-serine, in free amino acid form and/or in physiologically acceptable salt form in the preparation of a medicament or nutritional formulation with immune-modulating effect.

The invention also provides a method for effecting immune-modulation comprising administering to a human or other mammal in need of such a treatment a medicament or nutritional formulation comprising glycine, L-alanine and/or L-serine, in free amino acid form and/or in physiologically acceptable salt form said amount being effective to cause immune-modulation.

The term "amino acid of the invention" as used hereinafter is meant to refer to glycine, L-alanine and/or L-serine, in free amino acid form and/or in physiologically acceptable salt form.

The amino acid of the invention has an immune-modulating effect both in the natural defence against infection and in inflammatory processes.

The immune-modulation effected by the amino acid of the invention is not TNF-induced. This is shown by the fact that in burn patients or patients with ARDS (adult respiratory distress syndrome) TNF remains unchanged. This is also valid for models in which pathogenic bacteria are brought into interaction with human immune effective cells. Mediators other than TNF are affected in the presence of the amino acid of the invention suggesting its broad immune-modulatory capacity not related to TNF. In this regard human cells are primed by the amino acid of the invention for the release of interleukin-10 and $\gamma$-interferon.

The amino acid of the present invention has no influence on the immune system's bactericidal capabilities or on respiratory burst. These results indicate that the amino acid of the invention does not impair the phagocytic functions which kill ingested bacteria.

The amino acid of the present invention as a immune-modulator is performing the function of a primer of a weakened immune system and a damper of an exaggerated immune response and as such helps to reach homeostasis between a SIRS and a CARS (compensatory anti-inflammatory response syndrome). The resulting balance of the natural immunity is not dependent on a single mediator but rather on starting and keeping the balance of the mediator cascade.

The amino acids of the invention modulate the cellular signal transduction pathway. An interaction or participation of G-proteins leads to a modulation of the subsequent reply.

The invention further provides for the use of glycine, L-alanine and/or L-serine, a free amino acid form and/or in physiologically acceptable salt form in the preparation of a medicament or nutritional formulation which modulates leukotrienes, in particular activates $LTB_4$, increases interferon-$\gamma$- release and/or increases interleukin 10 (IL-10)-release. This is evident upon activation with a stimulus suggesting a priming role for the amino acid of the invention which stimulates host defence.

The present data show very clearly that the amino acid of the invention has immune-modulatory capabilities in the ex vivo- in vitro-model. With the chosen mediators, after activation of the cells with different activators, production of the oxygen radicals is not affected. Moreover, the present experiments show that the amino acid of the invention interacts with cell-biological signal points. There is a modulation of the NaF-induced mediator release. As is known, NaF is an activator which activates cells via G-proteins. The leukotriene release is modulated at salient points. Here, the affect on $LTE_4$ and $LTC_4$ should be mentioned in particular. The activation of $LBT_4$ is dependent on the type of activating stimulus. What is surprising with respect to cytokine release is the influence of the amino acid of the invention on the increased release of interferon-$\gamma$. This is observed especially if pre-incubation is effected with glycine and subsequent stimulation is carried out with SEB, that is the staphylococcus enterotoxin. As far as TNF release is concerned, modulation is dependent on the activating stimulus. The present results thus show very clearly that the amino acid of the invention has modulating properties on peripheral mononuclear cells, as well as on neutrophilic granulocytes. These modulating properties could be responsible for the beneficial effects of the amino acid of the invention in respect of stabilizing the defence against infections. The mediator release is modified, here in particular the interferon-$\gamma$ release is increased.

It is shown that essential salient points in the natural defence against infection are modulated in a striking manner. In this connection, the action of the amino acid of the invention in respect of interleukin-10 induction with variable activation of the lymphocytes, monocytes, basophils, is especially striking. There is a very significant increase in interleukin-10 release. As is known, interleukin-10 is an anti-inflammatory cytokine. Interleukin-10 acts on TH1-cytokine-releasing cells. Thus, the amino acid of the invention would be able to influence TH1-mediated disease processes. At this point, it must be noted that TH1-cytokines not only exert protective functions, but of course also deleterious functions within the limits of defence against infection. By taking the data together critically, the amino acid of the invention has no affect on inflammatory effector cells in respect of spontaneous activation of preformed mediators (e.g. enzymes, glucuronidase, histamine); the amino acid of the invention does not change the IgE induction protocol. With regard to its activity on cytokines, the amino acid of the invention has a suppressing effect on TNF release, and also under given conditions a stimulating effect on TNF release. With regard to the interleukin-10 release, under the chosen conditions, an increase in interleukin-10 takes place. Thus, the amino acid of the invention demonstrates marked immune-modulating activity, which could be of extreme importance concerning shock, sepsis and also allergically inflammable processes.

Conditions which can be treated with the amino acid of the present invention thus include inter alia shock, sepsis, allergic-inflammatory reactions and microbial infections whether they are caused by bacteria, parasites or viruses.

The nutritional formulation or medicament may be administered either prophylactically, e.g. preoperatively, in the acute phase, e.g. postoperatively, or both.

The nutritional formulation or medicament may be administered to the patient enterally or parenterally. The enteral administration route is preferred, particularly for subsequent or prophylactic treatment; particularly contemplated enteral administration routes are oral administration, nasal administration and/or tube feeding. The medicament or formulation is conveniently administered in the form of an aqueous liquid. The medicament or formulation in a form suitable for enteral application is accordingly preferably aqueous or in powder form, whereby the powder is conveniently added to water prior to use. For use in tube feeding, the amount of water to be added will depend, inter alia, on the patient's fluid requirements and condition. It will be appreciated that, for acute treatment, the parenteral application route is preferred. The parenteral application route is, for example, also indicated where the objective is to control the effects of chronic endotoxemia.

The medicament or formulation may be so formulated as to deliver to the patient from 1.5 to 80g of the amino acid of the invention per 24 hours. The amount of medicament or formulation to be administered depends to a large extent on the patients's specific requirements. Such daily amounts of amino acid of the invention are suitable for treatment of the desired effects as well as for prophylactic/pretreatment. In the case that the medicament or formulation comprises a single amino acid of the invention (in the L-configuration where applicable), it may be administered to the patient in an amount such that the concentration of that amino acid in the patients's plasma is elevated to between 0.3 and 2.0 mM, preferably from 0.8 to 2.0 mM. Whilst concentrations higher than this are anticipated, it is expected that significant clinical effects will be obtained if the concentration of the acid is increased, as a consequence of administration of the formulation or medicament, so that it lies in the range of from 0.8 to 1.5mM. In traumatic, hypercatabolic patients it may even be beneficial to raise the plasma glycine, L-serine or L-alanine levels to about 0.2 to 0.3 mM which corresponds to plasma glycine levels of healthy individuals.

The most preferred amino acid of the invention for incorporation into the medicament or formulation for use according to the invention is glycine or a physiologically acceptable salt thereof.

Generally, it is indicated to use an amino acid of the invention in combination with one or more of the following components:

(i) omega-3 polyunsaturated fatty acids (PUFAs) where desired in admixture with omega-6 PUFAs;

(ii) L-arginine or other physiologically acceptable compounds associated with the synthesis of polyamines, or mixtures thereof; and

(iii) a nucleobase source.

Whereby the use of a medicament or nutritional formulation comprising an amino acid of the invention in combination with arginine or other physiologically acceptable compounds associated with the synthesis of polyamines such as ornithine is preferred. Use of a medicament or nutritional formulation comprising an amino acid of the invention, arginine or ornithine and omega-3 polyunsaturated fatty acids (PUFAs) is also preferred.

Nucleobase sources suitable for use in combination with the amino acids of the invention comprise or consist of natural nucleobases, nucleosides, nucleotides, RNA, DNA, equivalents thereof and/of mixtures comprising one or more of these compounds.

Natural nucleobases include the purines adenine and guanine as well as the pyrimidines cytosine, thymine and uracil. Where the nucleobase source is in the form of free nucleobases, it is preferably uracil.

Natural nucleosides include the ribose nucleosides adenosine, guanosine, uridine and cytidine and the deoxyribose nucleosides deoxyadenosine, deoxyguanosine, deoxythymidine and deoxycytidine.

Natural nucleotides include phosphate esters of natural nucleosides, such as the monophosphates adenylate (AMP), guanylate (GMP), uridylate (UMP), cytidylate (CMP), deoxythymidiylate (dTMP), deoxycytidylate (dCMP), and diphosphates and triphosphates of natural nucleosides such as ADP and ATP.

A purified nucleobase source, such as yeast is preferred. However, other sources such as meat and the like may be used. Preferably the nucleobase source is RNA.

Accordingly, the invention provides medicaments or nutritional formulations comprising effective amounts of:

(a) an amino acid of the invention (component (a)) in association with one or more components selected from
(b) omega-3 PUFAs where desired in admixture with omega-6 PUFAs (component (b));
(c) L-arginine or other physiologically acceptable compounds associated with the synthesis of polyamines, or mixtures thereof (component (c)); and
(d) a nucleobase source (component (d)).

Said medicaments and nutritional formulations are hereinafter designated "diets of the inventions".

One unit dose of such a medicament or nutritional formulation preferably comprises 1.5 to 80 parts by weight of component (a) in association with the following amounts of one or more components selected from (b) to (d): 0.1 to 20 parts by weight of component (b), 3 to 40 parts by weight of component (c) and 0.1 to 4.0 parts by weight of component (d). Particularly preferred one unit dose comprises 1.5 to 80 parts by weight of component (a) in association with the following amounts of one or more components selected from (b) to (d): 2 to 5 parts by weight of component (b), 7.5 to 20 parts by weight of component (c) and 1.7 to 2.0 parts by weight of component (d).

The amount of components (a) to (d) administered daily will conveniently correspond to 1.5 to 80 g for component (a), 0.1 to 20 g, preferably 2 to 5 g, for component (b), 3 to 40 g, preferably 7.5 to 20 g, for component (c) and 0.1 to 4.0 g, preferably 1.7 to 2.0 g, for component (d).

With respect to component (d) the above dosage is indicated for RNA, DNA, nucleosides or nucleotides. For nucleobases one weight unit of nucleobases is regarded to be equivalent to 2.5 to 3.0 weight units of RNA, DNA, nucleosides or nucleotides.

Where medicaments or nutritional formulations comprising an amino acid of the invention in combination with one or more of the above-mentioned components (b), (c) and (d) are used, such medicaments or nutritional formulations will conveniently comprise in one unit dose

(a) 1.5 to 80 parts by weight of one or more amino acids selected from the group consisting of glycine, L-alanine and L-serine, in free form or physiologically acceptable salt form, or mixtures thereof,

in combination with one or more compounds selected from the group consisting of

(b) 2 to 5 parts by weight omega-3 polyunsaturated fatty acids;
(c) 7.5 to 20 parts by weight L-arginine or L-ornithine, or mixtures thereof; and
(d) 1.7 to 2.0 parts by weight RNA.

Preferred medicaments or nutritional formulations comprise in one unit dose:

(a) from 1.5 to 80 parts by weight of an amino acid selected from the group consisting of glycine, L-alanine and L-serine, in free form or physiologically acceptable salt form, or mixtures thereof, in association with
(c) 3 to 40 parts by weight, preferably 7.5 to 20 parts by weight, of arginine or an equivalent amount of one or more other physiologically acceptable compounds associated with the synthesis of polyamines, or an equivalent amount of a mixture of arginine with such compounds.

More preferably the medicaments or nutritional formulations of the invention comprise component (a) in combination with component (c) at a weight ratio of 1:2 to 4:1, particularly preferred at a weight ratio of 1:1 to 2:1.

Further preferred medicaments or nutritional formulations comprise in one unit dose:

(a) from 1.5 to 80 parts by weight of an amino acid selected from the group consisting of glycine, L-alanine and L-serine, in free form or physiologically acceptable salt form, or mixtures thereof, in association with
(b) 0.1 to 20 parts by weight, preferably 2 to 5 parts by weight, of omega-3 PUFAs; and
(c) 3 to 40 parts by weight, preferably 7.5 to 20 parts by weight, of L-arginine or an equivalent amount of one or more other physiologically acceptable compounds associated with the synthesis of polyamines, or an equivalent amount of a mixture of arginine with such compounds.

Omega-3 PUFAs are conveniently protected against peroxidation.

Physiologically acceptable ways of protecting omega-3 PUFAs against peroxidation are known in the art. They include physiologically acceptable micro-encapsulation of omega-3 PUFAs and the use of physiologically acceptable antioxidants.

A typical example suitable for use as physiologically acceptable micro-encapsulation agents is starch. The micro-encapsulation can be effected in a manner known per se. The micro-encapsules may be coated in a manner known per se, by physiologically acceptable coating agents such as Gum Arabic.

Typical examples of antioxidants suitable for use in the method of the invention include antioxidant vitamins such as Vitamin C, Vitamin E or mixtures thereof.

The amount of antioxydant added should be sufficient to prevent peroxidation of the omega-3 PUFAs. Such amounts can be easily calculated. In general, for convenience, any antioxydants employed to prevent peroxidation, will be employed in excess. It will be appreciated that the presence of any other agent administered in association with the omega-3 PUFAs may require adjustment of the amount of antioxidant to be employed.

The omega-3 PUFAs may be employed in a form suitable for the physiological supply of omega-3 PUFAs, e.g. in free acid form, in triglyceride form, or in the form of physiologically acceptable natural sources of omega-3 PUFAs. Such natural sources include linseed oil and fish oils such as menhaden oil, salmon oil, mackerel oil, tuna oil, codliver oil and anchovy oil. Said natural sources, in particular, the fish oils, comprise substantial amounts of omega-3 fatty acids. Where the omega-3 PUFAs are employed in triglyceride form, said triglycerides may comprise esters with other physiologically acceptable fatty acids. Preferred omega-3 PUFAs include eicosapentaenoic acid (EPA) and docosahexaenoic acid (DHA), in free acid form, in triglyceride form or in form of natural sources having a high EPA and/or DHA content.

When the amino acids of the invention are administered in the form of a medicament such a medicament will comprise from 1 to 99 g of the amino acid of the invention per 100g.

In general, favourable effects are obtained when administering the diets of the invention in the form of a formula diet, which may, depending on the circumstances be a complete formula diet (i.e. a diet supplying essentially all required energy, amino acids, vitamins, minerals and trace elements) or a diet supplement. The diet will conveniently be taken in aqueous liquid form. A formula diet accordingly may comprise a source of carbohydrates, lipids fat (fat source) and protein (nitrogen source), and at least one amino acid selected from the group consisting of glycine, L-alanine and L-serine, or physiologically acceptable salts thereof, characterized in that the acid or salt is present in the formula diet in an amount of about 0.5 to 10g per 100g. The formula diet will preferably further comprise other nutritionally advantageous components such as vitamins, minerals, trace elements, fibers (preferably soluble fibers).

Examples of suitable nitrogen sources include nutritionally acceptable proteins such as soy bean or whey derived proteins, caseinates, and/or protein hydrolysates. Suitable carbohydrate sources include sugars such as maltodextrins. Examples of suitable fat sources include triglycerides, as well as di- and monoglycerides.

Examples of vitamins suitable for incorporation into the medicament or formulation of the invention include Vitamin E, Vitamin A, Vitamin D, Vitamin K, folic acid, thiamin, riboflavin, Vitamin $B_1$, $B_2$, $B_6$ and $B_{12}$, niacin, biotin and panthotenic acid in nutritionally acceptable form.

Examples of mineral elements and trace elements suitable for incorporation into the medicament or formulation include sodium, potassium, calcium, phosphorous, magnesium, manganese, copper, zinc, iron, selenium, chromium, and molybdenum in nutritionally acceptable form.

In particular, the medicament or formulation will preferably comprise beta-carotene (Vitamin A), Vitamin E, Vitamin C, thiamine, Vitamin $B_{12}$, choline, selenium and zinc in nutritionally acceptable form.

The term "soluble fiber" as used herein refers to fibers which are able to undergo substantial fermentation in the colon ultimately to produce short chain fatty acids. Examples of suitable soluble fibers include pectin, guar gum, locust bean gum, xanthan gum which may optionally be hydrolysed. For adults the total amount of soluble fibre per day will conveniently lie in the range of from 3 to 30g.

It will be appreciated that omega-3 PUFAs may be administered in higher amounts than those indicated hereinabove, and that such higher amounts will in general not impair the desired effect or provoke undesired side effects.

Compounds particularly suitable for use as component (c) in the formulation of the invention include L-arginine and L-ornithine, most preferably L-arginine. Component (c) may be employed in free form, physiologically acceptable salt form, e.g. in the form of a salt with phosphoric acid, citric acid, tartaric acid, fumaric acid, adipic acid or lactic acid, or in small peptide form. Preferably L-arginine in free form is employed.

The term small peptides as used herein refers to peptides having from 2 to 6, preferably from 2 to 4 amino acids.

As already indicated, omega-3 PUFAs will conveniently be administered in the form of fish oils, protected or not against peroxidation. Such fish oils also comprises omega-6 PUFAs.

Omega-6 PUFAs have also a favourable effect on the immune response and on the resistance to infection upon surgery. Accordingly, diets of the invention will conveniently further comprise omega-6 PUFAs.

For the purpose of the invention the omega-6 PUFAs may be in free acid form or in a form suitable for the physiological supply of omega-6 PUFAs, e.g. in triglyceride form. Examples of omega-6 PUFAs particularly appropriate for use according to the invention, include linoleic acid and arachidonic acid, linoleic acid being most preferred. Examples of suitable omega-6 PUFA sources are known in the art. They include fish oils and vegetable oils. Examples of omega-6 PUFA sources having a high linoleic acid content such as safflower oil, sunflower oil, soya oil, cotton oil and corn oil.

Administration of a daily amount of omega-6 PUFAs in the range of from 1.5 to 5.0 g will in general suffice to attain a favourable effect. One unit dose of the medicaments or nutritional formulation defined above may accordingly further contain 1.5 to 5 parts by weight of omega-6 PUFAs.

In addition to components (b), (c) and (d), and omega-6 PUFAs further components may be added to the diets of the invention and may have a beneficial effect on the activity of the amino acid of the invention. An example of such beneficial components are omega-9 PUFAs. A preferred natural source for such fatty acid mixtures are fish oils. For taste and other reasons, the fish oils will, in oral application forms, preferably be used in encapsulated form.

Where the formula diet of the invention is intended for use as a nutritional supplement (e.g. pre-operative treatment), the amount of energy supplied by it should not be too excessive, in order not to unnecessarily suppress the patients appetite. The supplement should conveniently comprise energy sources in an amount supplying from 600 to 1000 Kcal/day. For use as a complete formula diet (e.g. for post-operative treatment, treatment of trauma), the diets of the invention will conveniently supply from 600 to 1500 Kcal/day. The contribution of the nitrogen source, carbohydrate source and lipid source to the total daily caloric may vary within wide ranges. In preferred formulations of the invention the carbohydrate source provides for 40 to 70 % of the total energy supply and, the nitrogen and fatty acid source each for 15 to 30 % of the total energy supply of the formulation. For use as complete diet, the diet of the invention will conveniently be administered in aqueous liquid form in volumes in the range of from 500 ml to 3000 ml. For use as a supplement, the administration may be in powder or liquid form.

Patients who can benefit from the present invention include e.g. trauma patients (polytrauma, burns, major surgery); patients with systemic inflammatory response syndrome (SIRS); septic patients; adult respiratory distress syndrome (ARDS) patients; patients at infection risk such as patients having a lowered resistance due to immunosuppression, patients subject to radio- and/or chemotherapy, patients suffering from diabetes mellitus, from protein-malnourishment, gastrointestinal cancer surgery patients, cardiac surgery patients, patients subject to transplantations, and patients suffering from human immunodeficiency virus-related infection; and patients before and following major operative procedures, i.e. any operative procedure requiring general anesthesia such as cardiac bypass surgery and major upper gastrointestinal surgery. As is shown herein, the amino acid of the invention is useful to stabilize innate immunity and to activate natural resistance.

The amino acids of the invention and especially the diets of the invention are as already set out above particularly suitable for treatment of patients due for surgery. Such pretreatment will be most effective when administering the diet of the invention in the form of a supplement. The supplement will advantageously be administered over a period of 3 days or longer. In general, a pretreatment starting 2 to 6 days before surgery, and during said 3-6 day period will be sufficient to attain the desired effect. For pretreatment or prophylactic purposes administration of a supplement containing from 1.5 g to 80 g amino acid of the invention in association with from 2 to 5 g Component (b) (omega-3-PUFAs) and/or with Component (c) supplying from 7.5 to 20 g L-arginine or L-ornithine per day, will in general give the desired effect.

Such a supplement may and preferably will also contain an effective amount of component (d), omega-6 PUFAs or further components as set out above.

The supplement will conveniently be administered in the form of unit doses suitable for administration of the supplement 1 to 4 times per day. Where the diets of the invention comprise energy sources, it is appropriate not to supply more than 1500 Kcal/day. Apart from this limitation with respect to the energy supply, diet supplements of the invention can and will conveniently be supplied in the form of complete formula diets as described above.

Typical administration forms suitable for such acute treatment are e.g. the aqueous solutions disclosed hereinbelow.

Typical pharmacologically acceptable formulation forms for oral administration will further comprise pharmacologically acceptable diluents, carriers, vitamins, spices, pigments and/or other adjuvants well known to the skilled person to be suitable for incorporation into such formulation.

The diets and formulations of the invention may be obtained in a manner known per se, e.g. by admixing the ingredients.

Typical formulations suitable for use according to the invention include aqueous solutions consisting essentially of 0.1 % to 90 % by weight of at least one amino acid selected from the group consisting of glycine, L-alanine and L-serine, and pharmaceutically acceptable salts thereof, the balance being distilled water. The amino acid of the invention may be present in a concentrated form of the solution in an amount of from 15 to 90% (by weight of the solution). Concentrated solutions are suitable for dilution to application forms or for use in acute treatment. Application forms having a lower content (e.g. 0.1 to 5 %) of the amino acid of the invention will in general be indicated for prophylactic purposes; concentrated forms of the solution having a higher content (e.g. 5 % to 40 % by weight) of amino acid of the invention will in general be more suitable for acute treatment.

Other formulations suitable for inclusion in the medicament or formulation of the invention, in particular for parenteral application, include infusion solutions such as Ringer's injection solution, lactated Ringer's injection solution, crystalloids, colloids or other plasma substitutes, in association or enriched with about 0.1 to 5.0g per liter infusion solution of glycine, L-serine and/or L-alanine. Ringer's injection solution is a sterile solution, containing from 3.23 to 3.54g of sodium (equivalent to from 8.2 to 9.0g of sodium chloride), from 0.149 to 0.165 of potassium (equivalent to from 0.285 to 0.315g of potassium chloride), from 0.082 to 0.098g of calcium (equivalent to from 0.3 to 0.36g of calcium chloride, in the form of $CaCl_2 \cdot 2H_2O$), from 5.23 to 5.80g of chloride (as NaCl, KCl and $CaCl_2 \cdot 2H_2O$) and water in sufficient quantity to give 1000 ml solution. Lactated Ringer's Injection solution is a sterile solution containing from 2.85g to 3.15g sodium, as chloride and lactate), from 0.141 to 0.173g of potassium (equivalent to from 0.27g to 0.33g of potassium chloride), from 0.049 to 0.060g calcium (equivalent to from 0.18g to 0.22g of $CaCl_2.2H_2O$), from 2.31g to 2.61g of lactate, from 3.68 to 4.08g of chloride (as NaCl, KCl and $CaCl_2 \cdot 2H_2O$) and water in sufficient quantity to give 1000 ml solution.

The terms crystalloids and colloids in connection with fluid therapy are known in the art. They include plasma substitutes such as Haemaccel (polygeline based) and Gelofusine (gelatin based).

The invention will be further understood by reference to the following specific description. The relevance of the following in vitro examples for the in vivo effects claimed is demonstrated e.g. in Eicosanoids (1990) 3; 1-22 "The neutrophil and leukotrienes - role in health and disease" by W. König et al. or in The Journal of Trauma (1990) 30(1); 1372-1379 "Basophil Releasability in Severly Burned Patients" by U. Bergman et al.

EXAMPLES

*Example 1. Investigation of respiratory burst:*

Human neutrophils (PMN) are isolated from EDTA-anticoagulated blood by Ficoll-density-centrifugation and by dextran sedimenation as described by Böyum, A. in Scand. J. Clin. Lab. Invest. 21(Suppl. 97):77-89, 1968. Variable concentrations of glycine (4.0 mM, 0.4 mM and 0.04 mM in a volume of 50 µl) and as a control 50 µl PBS are incubated with human neutrophils ($2 \times 10^7$ cells/ml in a volume of 500 µl in Ca/Mg (1/0.5mM) and 0.25mM luminol) for 20 min at 37°C and stimulation is subsequently carried out with fMLP (N-formyl-methionyl-leucyl-phenylalanine, $10^{-5}$M), NaF (20mM) or PMA (10 ng), each in a volume of 50µl, for 30 min at 37°C. fMLP is a receptor-mediated stimulus, NaF is a G-protein-mediated activator of cells and PMA activates the protein kinase C. As a control neutrophils are incubated with the variable glycine concentrations without subsequent stimulation. Respiratory burst is measured by chemiluminescence response of oxygen radicals essentially as described in Immunology, 1992, 76, 86-94.

It was found that variable glycine concentrations have no effect on the oxygen metabolites. If stimulation is carried out with fMLP, there is a different response in the presence of fMLP. A partial time-dependent suppression of the respiratory burst is obtained, with the maximum however being reached later. At a concentration of 0.04 mM, there is a further modulation of the respiratory burst. If stimulation is carried out with PMA; here also, as time progresses, there is an increase in the respiratory burst in the presence and absence of glycine. No changes can be seen. If the cells are stimulated with NaF, a dose-dependent suppression and also an increase in respiratory burst takes place. These results show that glycine clearly modulates signal transduction elements on interaction with the cells, so that the following NaF stimulus leads either to suppression or an increase in respiratory burst. With fMLP there is hardly any change in the respiratory burst. If these experiments are summarized, they prove that the ability of the neutrophil to produce oxygen radicals is not modulated by glycine. Moreover, glycine probably interacts with the cellular signal transduction tract, especially as the subsequent stimulation with NaF leads to a modulation of the respiratory burst. These experiments verify that glycine clearly modulates the cellular signal transduction cascade. Through the interaction or participation of G-proteins, there is a modulation of the subsequent response.

### Example 2. Investigation of leukotriene generation

Neutrophils (PMNs, isolated as described in example 2, in a concentration of $2 \times 10^7$/ml in a volume of $500 \mu l + 50 \mu l$ 1mM Ca/0.5mM Mg) are preincubated with glycine (0.0004mM, 0.004mM, 0.04mM, 0.4mM and 4mM in a volume of $50 \mu l$) for 20 or 30 minutes at 37°C. Subsequently they are treated with cytochalasin ($50 \mu l$, $10^{-5}$M) and stimulated with $50 \mu l$ fMLP ($10^{-5}$M), NaF (20mM), or primed with $50 \mu l$ GM-CSF (10ng) and subsequently stimulated with $50 \mu l$ NaF (20mM), fMLP ($10^{-5}$M) or Ca-ionophore ($5 \mu M$) and incubated for a further 20 minutes at 37°C. Leukotrienes are measured by HPLC as described in Immunology 67:401-407.

Leukotrienes are lipid mediators; they are obtained from arachidonic acid via 5-lipoxygenase activation. They display a chemotactic or spasmogenic activity profile. They take part in cellular interaction processes and stabilize or modulate the cytokine-induced immune response. Activators for leukotriene generation are the calcium ionophore, the bacterial peptide fMLP or the G-protein activator NaF. What is striking is the suppressing influence of glycine at a concentration of 4.0 mM on the fMLP-induced $LTE_4$ generation. Pre-incubation over 30 minutes similarly led to a suppression of the $LTC_4$ release, while the $LTB_4$ release as well as the omega-$LTB_4$ release experienced an increase. The activation of neutrophils in the presence of glycine with the calcium ionophore similarly shows a very strong suppression for leukotriene-$E_4$. With fMLP, there is similarly a suppression in the course of $LTB_4$ generation. Continuing experiments were then carried out, whereby peripheral mononuclear cells were primed with GM-CSF (granulocyte/macrophage-colony-stimulating factor) and subsequently incubated with variable concentrations of glycine and then activated with calcium ionophore, fMLP or NaF. For the calcium ionophore, there is a marginal increase in $LTB_4$ generation; for NaF there is an inhibition of $LTC_4$ generation and also $LTB_4$ generation, and for fMLP there is a fluctuating $LTB_4$ release. By combining the previous experiments, for leukotriene generation an effect is shown on leukotriene-$E_4$ and also leukotriene-$C_4$. Furthermore, it is evident that glycine clearly interacts with cells by means of signal transduction tracts, so that the subsequent activation of the 5-lipoxygenase sequence is modulated. Here also, glycine has immune-modulatory characteristics.

### Influence of glycine on superantigen primed neutrophils

Neutrophils (PMNs, isolated as described in example 1, in a concentration of $2 \times 10^7$/ml in a volume of $500 \mu l + 50 \mu l$ 1mM Ca/0.5mM Mg) were primed with $50 \mu l$ microbial superantigens (staphylococcus enterotoxin B, staphylococcus enterotoxin B-T-cell receptor mutant, staphylococcus enterotoxin B MHC-mutant, staphylococcus enterotoxin A, staphylococcus enterotoxin A-T-cell receptor mutant) as well as recombinant toxic shock syndrome toxins P17 and P135 and incubated for 10 minutes at 37°C. The incubation with various concentrations of glycine (40 $\mu M$, 0.4M and 4mM in a volume of $50 \mu l$) and a PBS control was then carried out for a further 10 minutes, and the subsequent stimulation with $50 \mu l$ fMLP ($10^{-5}$M) continued for a further 20 minutes. The superantigens were employed in a concentration of 1 ng/ml The supernatants of stimulated cells were analyzed for 12-HETE as described previously in J. Chromatogr. 427:199-208, 1988 and Infect. Immun. 58:1591-1599. The following results were obtained:

There is an increase in omega-$LTB_4$ generation, and in particular the release of 12-HETE (12 hydroxyeicosatetraenoic acid). In the presence of staphylococcus enterotoxin B there is similarly an increase in the cysteinyl leukotrienes and a marginal increase in 12-HETE. In the presence of TSST, there is a dose-dependent increase in 20-hydroxy-$LTB_4$ with a drop in 20-carboxy-$LTB_4$; furthermore, there is an increase in $LTC_4$, $LTE_4$, and also a dose-dependent increase in 12-HETE; with the TSST-1-mutant P135, the increase in cysteinyl leukotrienes and in 12-HETE is marginal; pre-incubation with staphylococcus enterotoxin A leads to a marked increase in 20-hydroxy-$LTB_4$, a drop in $LTE_4$, a marked increase in 12-HETE, and the SEA-T-cell receptor mutant marginally shifts this image (SEA = staphylococcus enterotoxin A). The SEB-T-cell receptor mutant leads to a significant drop in 20-carboxy-$LTB_4$, a marginal increase in 12-HETE, while the SEB-MHC-mutant leads to a drop in 20-carboxy-$LTB_4$. These results show that pre-treatment of neutrophil granulocytes with superantigens in recombinant form, and also with the superantigen molecules as mutants, have a modulating effect on the subsequent incubation with glycine and stimulation with fMLP. This modulation normally leads to a shift in $LTB_4$ metabolisation with an increase in 20-hydroxy-$LTB_4$, which is more inactive biologically than the leukotriene $B_4$. The increase in 20-hydroyeicosatetraenoic acid, which has chemotactic activity, and also in the cysteinyl leukotrienes, may be of great significance to vascular regulation. Overall, these findings however indicate an immune-modulatory role of "glycine" in terms of a potential reactivation of suppressed cellular functions.

### Example 3. Investigation of cytokine release

### Influence of glycine on interleukin-8 release

Peripheral mononuclear cells (PMNs, isolated as described in example 1, in a concentration of $2 \times 10^7$/ml in a volume of 500µl + 50 µl 1mM Ca/0.5mM Mg) are treated with variable concentrations of glycine (0.0004mM, 0.004mM, 0.04mM and 0.4mM in a volume of 50µl) and optionally with interferon-$\gamma$ and incubated for 30 min at 37°C and subsequently incubated with cytochalasin B (50 µl, $10^{-5}$M) and stimulated with 50 µl fMLP ($10^{-5}$M). Interleukin-8 as a chemotactically active cytokine, as well as the tumour necrosis factor TNF, are analysed. Apart from stimulation with fMLP, RSV (respiratory syncytial virus), phospholipase C and SEB are also used as activators. IL-8 release was determined by a sandwich ELISA technique as described in Int. Arch Allergy Immunol 1995; 106:357-365. TNF release was determined by a sandwich ELISA technique established by the company Genzyme (Human TNF-$\alpha$: DuoSet®).

There is a significant increase in IL-8 release after stimulation with RSV at 60 mins. and pre-incubation with glycine. These experiments also verify that glycine interacts with the cellular signal transduction cascade. The subsequent stimulation with RSV, which similarly progresses via G-proteins, leads to an increased release of interleukin-8. Under the conditions selected, there are no significant changes in respect of TNF release. In the fMLP induced stimulation batches, no effect is observed as to interleukin-8 release. With staphylococcus enterotoxin B there is a marginal increase in interleukin-8 release. If the cells are pre-incubated with interferon-$\gamma$ and subsequently stimulated with phospholipase C or with staphylococcus enterotoxin B, a dose-dependent stimulation of interleukin-8 release is obtained with phospholipase C and also with staphylococcus enterotoxin B. Taken overall, the findings on interleukin-8 release are not so convincing. Marginal effects are observed regarding the glycine-induced modulation of interleukin-8 release in the presence of different activating stimuli.

### Investigation of interleukin-10 release

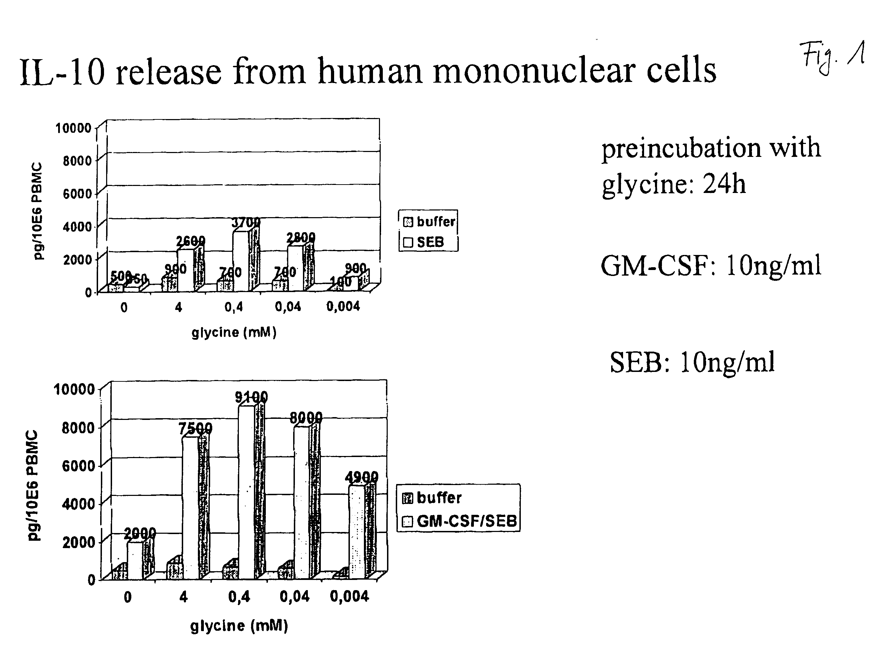

In the following, lymphocytes, monocytes, basophils (LMBs, obtained as described in Scand. J. Lab. Invest., 97(Suppl): 77-89, 1968, at a concentration of $4 \times 10^6$ or $2 \times 10^7$ml in a volume of 500µl and 50µl 1mM Ca/0.5mM Mg) are either primed with 50 µl GM-CSF (10 ng) or PBS for 30 min at 37°C, then incubated with variable concentrations of glycine (4.0 mM - 4.0 µM) and a PBS control for a further 30 min. and then incubated with 50 µl fMLP ($10^{-5}$M) in the presence of cytochalasin B (50 µl, $10^{-6}$M) or with PBS for 24-48 hours. In this batch, there is hardly any change in interleukin-10 release. The same applies when staphylococcus enterotoxin B (50µl SEB, 10 ng) or RSV are employed instead of fMLP in the presence of cytochalasin B. The same applies to the pre-incubation with glycine; however during subsequent stimulation with SEB (10ng), there is an increase in interleukin-10. A dose-dependent effect is also observed after stimulation with RSV. This increase in interleukin-10 release is also found after priming of the cells with GM-CSF. A dose-dependent increase is similarly observed after incubation with phospholipase C. Our findings show very clearly that glycine combined with staphylococcus enterotoxin B leads to a significant release of interleukin-10 (see Fig. 1). Interleukin-10 is a cytokine having anti-inflammatory activity. One might imagine that this is one of the essential principles of action of glycine in respect of the suppressing and modulating function.

### Investigation of TNF release

Peripheral mononuclear cells (PMNs, isolated as described in example 1, in a concentration of $2 \times 10^7$/ml in a volume of 500µl + 50 µl 1mM Ca/0.5mM Mg) were pre-incubated with glycine at variable concentrations (0.0004mM, 0.004mM, 0.04mM and 0.4mM in a volume of 50µl) and a PBS control and subsequently stimulated with SEB (10 ng). There is a dose-dependent increase in TNF. In the GM-CSF (10 ng) primed model, the following SEB (10 ng) stimulation leads to a suppression of the TNF release. Pre-incubation of the cells with glycine and subsequent stimulation with SEB similarly leads to a suppression of TNF release in dependence on stimulus. Pre-incubation of the cells with glycine and subsequent stimulation with SEB leads to an increase in SEB-induced TNF release. A modulation of TNF release is similarly observed if RSV is used as stimulus. This also applies to the bacterial toxin, phospholipase C, which was used at variable concentrations.

## Investigation of TNF-$\alpha$ release

In the following, lymphocytes, monocytes, basophils (LMBs, obtained as described in Scand. J. Lab. Invest., 97(Suppl): 77-89, 1968, at a concentration of $10^6$/ml) were preincubated for 2 hours with variable concentrations of glycine as in the previous examples and subsequently stimulated with SEB (10 ng). In contrast to the stimulated control without glycine, there is great suppression of the TNF-$\alpha$ release . This is similarly noticed if cells are firstly primed with 10 ng GM-CSF for 24 hours; glycine incubation is then carried out for 2 hours and stimulation follows with staphylococcus enterotoxin B. There is similarly an inhibition of TNF-$\alpha$ release. If the cells are firstly incubated with glycine for 2 h and the GM-CSF addition (24 h incubation) and stimulation with SEB follows, there is similarly a marked suppression of the SEB-induced TNF-$\alpha$ release. Continuing experiments were carried out in order to change the pre-incubation time of glycine. Peripheral lymphocytes, monocytes, basophils (LMBs) were pre-incubated with glycine for 24 hours and subsequently incubated with staphylococcus enterotoxin B. An increase in TNF-$\alpha$ release takes place, compared with the control. This increase is again suppressed if first of all GM-CSF priming had taken place and subsequently stimulation is effected with staphylococcus enterotoxin B. Continuing changes to the incubation protocol relate to the pre-incubation time. The pre-incubation time for glycine was restricted to 2 hours, then priming took place with GM-CSF for 24 hours and stimulation with staphylococcus enterotoxin B for a further 24 hours; an increase in TNF-$\alpha$ takes place. This increase is similarly noticeable if pre-incubation with GM-CSF was effected, then incubation was carried out with glycine and a further priming with GM-CSF was carried out over a further 24 hours. These results very significantly emphasize the modulatory influence of glycine which changes in dependence on the pre-incubation time and under the chosen conditions of GM-CSF. What is striking is the marked suppression of TNF-$\alpha$ release in the presence of glycine and the staphylococcus enterotoxin B. In addition, what is also remarkable is that priming of the cells with GM-CSF and stimulation with glycine lead to an increase in cellular activation. Glycine thus appears to exert dual functions; on the one hand it suppresses the TNF-$\alpha$ release, and on the other hand, as a modulator, it also encourages immune stimulation and activation of TNF. As is known, TNF is an important mediator in immune regulation. A long-term reduction of TNF leads to the tackling of opportunistic infections.

In continuing investigations, the lymphocytes, monocytes, basophils (LMBs) were incubated with interferon-$\gamma$ for 24 hours. The cells were subsequently stimulated with staphylococcus enterotoxin B or phospholipase C in concentrations of 0.5 units/ml and also 0.1 units/ml. In many systems, interferon-$\gamma$ leads to regulation of TNF-$\alpha$ release. In these systems, there is a great suppression of the staphylococcus enterotoxin B and also phospholipase C induced TNF-$\alpha$ release by glycine. This suppression of the TNF-$\alpha$ release is lower if LMBs are previously pre-incubated with GM-CSF and subsequently stimulated with phospholipase C. Clearly, there is a protective effect of the GM-CSF towards the phospholipase C and in the glycine induced TNF-$\alpha$ suppression.

## Interleukin-12.

Interleukin-12 as a cytokine has pleiotropic properties. In the course of defence against infections, as the TH1-cytokine, it has activating functions on macrophages. In addition, of course, negative functions of interleukin-12 are also known, especially in the case of auto-immune illnesses. Here, there is also the notional concept that a mediator has dual biological functions. First of all, we looked into the question regarding to what extent glycine incubation of LMBs leads to interleukin-12 release. With low concentrations of glycine, a stimulatory effect of interleukin-12 is found. In the presence of staphylococcus enterotoxin B, there is a marginal reduction in interleukin-12 release if cells were primed with GM-CSF and subsequently preincubation with glycine was carried out for 2 hours, followed by stimulation with SEB. Here also, there is suppression of the interleukin-12 release, which is eliminated with low concenrations of glycine. The results thus indicate very clearly that glycine also exerts modulatory functions in respect of interleukin-12. IL-12 was determined by ELISA Sandwich technique as established by M. Gately (Hoffmann-La Roche, Nutley, New Jersey, USA)

## Investigation of inteferon-$\gamma$

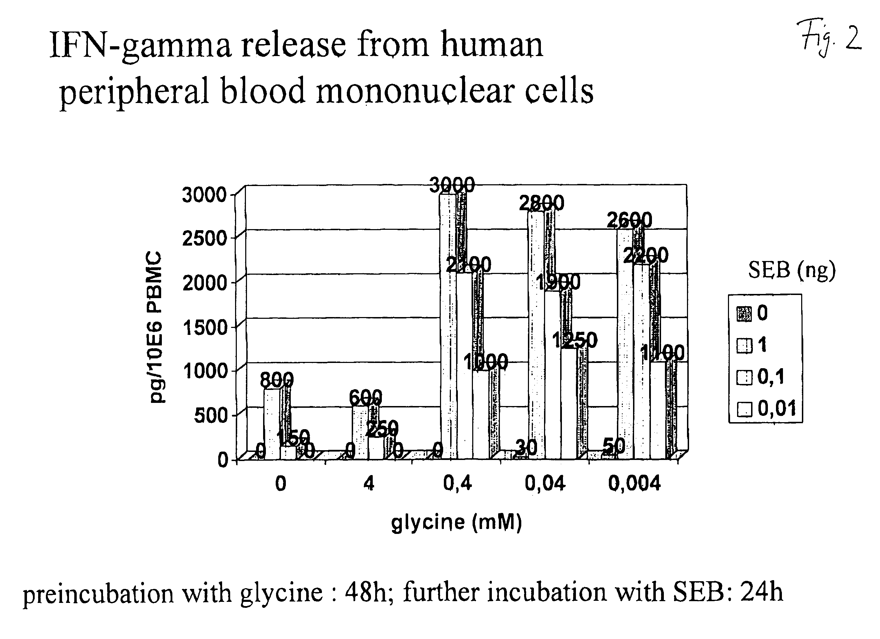

Peripheral mononuclear cells (as in example 2 but in a concentration of $10^6$/ml) are pre-incubated with SEB at variable concentrations (50 $\mu$l of 1 ng/ml, 0,1 ng/ml and 0.01 ng/ml) in the presence of variable concentrations of glycine (as above) for 24h, 48h and 72h or pre-incubated with glycine and subsequently stimulated with different concentrations of SEB. Marginal effects of interferon-$\gamma$ release take place with incubation of up to 48 hours. An increase in interferon-$\gamma$ release takes place with glycine pre-incubation over 72 hours if SEB is used as activator. If the cells are pre-incubated with glycine and subsequently stimulated with SEB, there is an extremely potent release of interferon-$\gamma$ after SEB stimulation. A very long pre-incubation with glycine and subsequent stimulation with interferon-$\gamma$, moreover, leads to a reduction in the interferon-$\gamma$ concentration. If RSV is used instead of SEB, a dose-dependent increase in interferon-$\gamma$ release takes place. This also applies to pre-incubation of the cells. There is intense increase of the interferon-$\gamma$ release

after 48 hours pre-incubation with glycine and subsequent stimulation with RSV. This is similarly indicated for the subsequent SEB stimulation (see Fig. 2).

### Example 4. Influence of glycine on Ig-synthesis.

To this end, peripheral mononuclear cells (as in example 1) were incubated with variable concentrations of glycine (0.00004mM, 0.0004mM, 0.004mM, 0.04mM, 0.4mM, 4mM, 40mM). The incubation time is carried out over 14 days. The cells were stimulated in the presence of glycine with interleukin-4 and anti-CD40 as B-cell activator. In the supernatant, the IgM, IgA, IgG and IgE synthesis was analysed. Although the interleukin-4 anti-CD40 system is a very strong activator of IgE synthesis, glycine has no affect on the induced IgE synthesis. Similarly, no changes are found in respect of the other immunoglobulins. To summarize these findings, glycine does not particularly favour IgE induction.

### Example 5: Enteral Compositions

In the following compositions MM stands for "mineral mixture", SM for "trace element mixture" and VM for "vitamin mixture". The composition of these three mixtures is as follows:

## MM

| Ingredients | g/100g |
|---|---|
| Maltodextrins | 34.40 |
| Potassium citrate/phosphate | 34.60 |
| Magnesium dicitrate | 8.20 |
| Calcium chloride | 8.00 |
| Sodium citrate/chloride | 9.00 |
| Citric acid | 3.50 |
| Choline tartrate | 2.30 |

## SM

| Ingredients | g/100g |
|---|---|
| Maltodextrins | 47.79 |
| Molybdenum-yeast | 18.00 |
| Chromium-yeast | 9.20 |
| Zinc sulfate | 7.00 |
| Selenium-yeast | 7.00 |
| Ferrum(II) sulfate | 6.92 |
| Copper(II) gluconate | 2.24 |
| Manganese(II) sulfate | 1.12 |
| Sodium fluoride | 0.70 |
| Potassium iodide | 0.03 |

## VM

| Ingredients | g/100g |
|---|---|
| Maltodextrins | 43.44 |
| Sodium ascorbate | 35.00 |
| Vitamin E-Ac. 50% | 16.00 |
| Niacinamide | 1.55 |
| Vitamin A-Acetate | 1.20 |
| Ca-D-Panthothenat | 0.98 |
| Vitamin $K_1$ 1% | 0.71 |
| Vitamin $B_{12}$ 0.1% | 0.30 |
| Vitamin $D_3$ | 0.28 |
| Vitamin $B_6$ | 0.20 |
| Vitamin $B_1$ | 0.17 |
| Vitamin $B_2$ | 0.15 |
| Folic acid | 0.02 |
| Biotin | 0.01 |

| Composition Comprising Glycine | |
|---|---|
| Ingredients | g/100g |
| Water | 77.40 |
| Maltodextrins | 10.10 |
| Na/Ca caseinates | 4.60 |
| Glycine | 3.00 |
| MM | 2.00 |
| SM | 0.05 |
| VM | 0.10 |
| β-Carotine | 0.03 |
| Lipids: | |
| Palm oil | 2.33 |
| Sunflower oil | 0.26 |
| Emulsifier Nathin E | 0.13 |
| | $\overline{100.00}$ |

| Composition Comprising Glycine and Arginine | |
|---|---|
| Ingredients | g/100g |
| Water | 77.40 |
| Maltodextrins | 8.93 |
| Na/Ca caseinates | 4.60 |
| Glycine | 3.00 |
| L-Arginine | 1.17 |
| MM | 2.00 |
| SM | 0.05 |
| VM | 0.10 |
| β-Carotine | 0.03 |
| Lipids: | |
| Palm oil | 2.36 |
| Sunflower oil | 0.23 |
| Emulsifier Nathin E | 0.13 |
| | $\overline{100.00}$ |

| Composition Comprising Glycine and Fish Oil (ω-3 fatty acids) | |
|---|---|
| Ingredients | g/100g |
| Water | 77.40 |
| Maltodextrins | 10.10 |
| Na/Ca caseinates | 4.60 |
| Glycine | 3.00 |
| MM | 2.00 |
| SM | 0.05 |
| VM | 0.10 |
| β-Carotine | 0.03 |
| Lipids: | |
| Palm oil | 1.32 |
| Sunflower oil | 0.23 |
| Emulsifier Nathin E | 0.13 |
| Fish Oil EPAX 3000 TG | 1.04 |
| | 100.00 |

| Composition Comprising Glycine and RNA | |
|---|---|
| Ingredients | g/100g |
| Water | 77.40 |
| Maltodextrins | 9.96 |
| Na/Ca caseinates | 4.60 |
| Glycine | 3.00 |
| Yeast extract RNA | 0.14 |
| MM | 2.00 |
| SM | 0.05 |
| VM | 0.10 |
| β-Carotine | 0.03 |
| Palm oil | 2.33 |
| Sunflower oil | 0.26 |
| Emulsifier Nathin E | 0.13 |
| | 100.00 |

| Composition Comprising Glycine, Arginine and Fish Oil ($\omega$-3 fatty acids) | |
|---|---|
| Ingredients | g/100g |
| Water | 77.40 |
| Maltodextrins | 8.93 |
| Na/Ca caseinates | 4.60 |
| Glycine | 3.00 |
| L-Arginine | 1.17 |
| MM | 2.00 |
| SM | 0.05 |
| VM | 0.10 |
| $\beta$-Carotine | 0.03 |
| Lipids: | |
| Palm oil | 1.32 |
| Sunflower oil | 0.23 |
| Emulsifier Nathin E | 0.13 |
| Fish Oil EPAX 3000 TG | 1.04 |
| | 100.00 |

| Composition Comprising Glycine, Arginine and RNA | |
|---|---|
| Ingredients | g/100g |
| Water | 77.40 |
| Maltodextrins | 8.79 |
| Na/Ca caseinates | 4.60 |
| Glycine | 3.00 |
| L-Alginine | 1.17 |
| Yeast extract RNA | 0.14 |
| MM | 2.00 |
| SM | 0.05 |
| VM | 0.10 |
| $\beta$-Carotine | 0.03 |
| Lipids: | |
| Palm oil | 2.33 |
| Sunflower oil | 0.26 |
| Emulsifier Nathin E | 0.13 |
| | 100.00 |

| Composition Comprising Glycine, RNA and Fish Oil ($\omega$-3 fatty acids) | |
|---|---|
| Ingredients | g/100g |
| Water | 77.40 |
| Maltodextrins | 9.96 |
| Na/Ca caseinates | 4.60 |
| Glycine | 3.00 |
| Yeast extract RNA | 0.14 |
| MM | 2.00 |
| SM | 0.05 |
| VM | 0.10 |
| $\beta$-Carotine | 0.03 |
| Lipids: | |
| Palm oil | 1.32 |
| Sunflower oil | 0.23 |
| Emulsifier Nathin E | 0.13 |
| Fish Oil EPAX 3000 TG | 1.04 |
| | 100.00 |

| Composition Comprising Glycine, Arginine, RNA and Fish Oil ($\omega$-3 fatty acids) | |
|---|---|
| Ingredients | g/100g |
| Water | 77.40 |
| Maltodextrins | 8.79 |
| Na/Ca caseinates | 4.60 |
| Glycine | 3.00 |
| L-Arginine | 1.17 |
| Yeast extract RNA | 0.14 |
| MM | 2.00 |
| SM | 0.05 |
| VM | 0.10 |
| $\beta$-Carotine | 0.03 |
| Lipids: | |
| Palm oil | 1.32 |
| Sunflower oil | 0.23 |
| Emulsifier Nathin E | 0.13 |
| Fish Oil EPAX 3000 TG | 1.04 |
| | 100.00 |

As already set out above, fish oil is a natural source for omega-3 PUFAs whereas sunflower oil is a natural source for omega-6 PUFAs.

**Claims**

1. The use of glycine, L-alanine and/or L-serine, in free amino acid form and/or in physiologically acceptable salt form in the preparation of a medicament or nutritional formulation with immune-modulating effect.

2. The use of glycine, L-alanine and/or L-serine, in free amino acid form and/or in physiologically acceptable salt form in the preparation of a medicament or nutritional formulation which modulates leukotrienes, in particular activates $LTB_4$, increases interferon-$\gamma$- release and/or increases interleukin 10 (IL-10)-release.

3. The use according to claim 2 wherein the medicament or nutritional formulation modulates leukotrienes, in particular activates $LTB_4$.

4. The use according to claim 2 wherein the medicament or nutritional formulation increases interferon-$\gamma$-release.

5. The use according to claim 2 wherein the medicament or nutritional formulation increases interleukin 10 (IL-10)-release.

6. The use of glycine, L-alanine and/or L-serine, in free amino acid form and/or in physiologically acceptable salt form in the preparation of a medicament or nutritional formulation which stabilizes the host defence against infection and activates natural resistance of the host.

7. The use of any one of the preceding claims wherein the medicament or nutritional formulation provides a dosage of 1.5 to 80 g of glycine, "L-alanine and/or L-serine, in free amino acid form and/or in physiologically acceptable salt form per 24 hours.

8. The use of any one of the preceding claims wherein the medicament or nutritional formulation further comprises one or more components selected from

    (i) omega-3 polyunsaturated fatty acids (PUFAs) where desired in admixture with omega-6 PUFAs;

    (ii) L-arginine or other physiologically acceptable compounds associated with the synthesis of polyamines, or mixtures thereof; and

    (iii) a nucleobase source.

9. The use of any one of the preceding claims wherein the medicament or nutritional formulation is useful for the treatment of allergic-inflammatory reactions.

# IL-10 release from human mononuclear cells

Fig. 1

preincubation with
glycine: 24h

GM-CSF: 10ng/ml

SEB: 10ng/ml

EP 0 855 181 A2

# IFN-gamma release from human peripheral blood mononuclear cells

Fig. 2

EP 0 855 181 A2

preincubation with glycine : 48h; further incubation with SEB: 24h